# EUROPEAN PATENT APPLICATION

(11) **EP 4 119 565 A1**
(43) Date of publication of application: **18.01.2023**
(21) Application number: 21767654.3
(22) Date of filing: 10.03.2021
(51) Int. Cl.: C07D 513/04, A61K 31/54, A61P 29/00, A61P 37/06, A61P 25/00, A61P 19/02, A61P 13/12, A61P 19/06, A61P 11/00, A61P 9/00, A61P 9/10, A61P 3/10, A61P 31/04, A61P 19/10, A61P 15/00, A61P 15/02, A61P 27/02, A61P 27/06, A61P 3/04, A61P 21/00, A61P 17/00

(54) **JAK KINASE INHIBITOR AND PREPARATION AND APPLICATION THEREOF**

(30) Priority: 10.03.2020 CN 202010162689
(71) Applicant: Minghui Pharmaceutical (Shanghai) Limited, Pilot Free Trade Zone Pudong New Area Shanghai 201203 (CN); Minghui Pharmaceutical (Hangzhou) Limited, Hangzhou, Zhejiang 310018 (CN)
(72) Inventor: LI, Ao, Shanghai 201203 (CN); JADHAV, Prabhakar Kondaji, Shanghai 201203 (CN); YAO, Yuanshan, Shanghai 201203 (CN); CHEN, Yile, Shanghai 201203 (CN); CAO, Guoqing, Shanghai 201203 (CN)
(74) Representative: Lavoix
(86) International application number: PCT/CN2021/080080
(87) International publication number: WO 2021/180143

(57) **Abstract**

The present invention provides a JAK kinase inhibitor and a preparation and application thereof, and the present invention specifically provides a compound having a structure represented in the following formula I, an enantiomer thereof, or a pharmaceutically acceptable salt thereof. The compound has uniquely advantageous JAK kinase inhibitory activity, and as such may be used for treatment of a disease or illness related to JAK kinase activity or expression level.

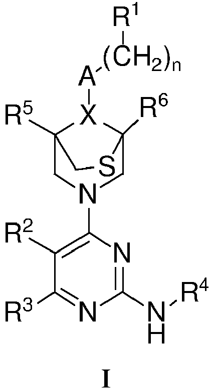

## Description

### TECHNICAL FIELD

The present invention relates to the field of pharmaceutical compound, in particular, the present invention provides a JAK kinase inhibitor and the preparation and application thereof.

### BACKGROUND OF THE INVENTION

Protein kinases are a family of enzymes that catalyze the phosphorylation of specific residues in proteins and are generally divided into tyrosine kinases and serine/threonine kinases. Abnormal kinase activity lead by mutation, overexpression or inappropriate regulation and excessive or insufficient production of growth factors or cytokines is associated with many diseases, including but not limited to cancers, cardiovascular diseases, allergies, asthma and other respiratory diseases, autoimmune diseases, inflammatory diseases, skeletal diseases, metabolic diseases, and neurological and neurodegenerative diseases (e.g., Alzheimer's disease). Inappropriate levels of in vivo kinase activity trigger a variety of biological cellular responses which relates to cell growth, cell differentiation, cell function, survival, apoptosis and cell migration associated with the aforementioned diseases.

Therefore, protein kinases have become an important class of enzymes as targets for therapeutic intervention. In particular, the JAK family of cellular protein tyrosine kinases plays a central role in cytokine signaling. Upon binding to their receptors, cytokines activate JAK, which in turn phosphorylate cytokine receptors, creating docking sites for signaling molecules (especially members of the Signal Transducers and Activators of Transcription (STAT) family), eventually lead to gene expression. Therefore, compounds that potently and highly selectively inhibit specific JAK kinases may serve as potential therapeutics for a range of diseases or disorders, particularly inhibitors of TYK2 and JAK1.

TYK2 is a member of the JAK kinase family and is important in the signaling of type I interferons (IFNa, INFb), IL-6, IL-10, IL-12 and IL-23. Thus, TYK2 signals with other members of the JAK kinase family in the following combinations:
TYK2/JAK1, TYK2/JAK2, TYK2/JAK1/JAK2. TYK2 has shown importance in the differentiation and function of multiple cell types important for inflammatory and autoimmune diseases, including natural killer cells, B and T helper cell types.

JAK1 is expressed at different levels in all tissues. Many cytokine receptors signal through pairs of JAK kinases in the following combinations: JAK1/JAK2, JAK1/JAK3, JAK1/TYK2 or JAK2/JAK2. In this context, JAK1 is the most broadly paired JAK kinase and is required for signaling by gamma-common (IL-2Ry) cytokine receptor, including IL-6 receptor family, I, II and III receptor families, and IL-10 receptor family. Animal studies have shown that JAK1 is necessary for immune system development, function and homeostasis. Modulation of immune activity by inhibiting JAK1 kinase activity is proved useful in the treatment of various immune diseases.

In conclusion, there is still a need in the art to develop inhibitors against JAK family protein kinases, especially TYK2 or JAK1 protein kinases.

### SUMMARY OF THE INVENTION

The object of the present invention is to provide an inhibitor against JAK family protein kinase inhibitors, especially TYK2 and/or JAK1 protein kinase inhibitors.

In a first aspect of the present invention, it provides a compound of the structure shown in formula I, or an enantiomer, or a pharmaceutically acceptable salt thereof: 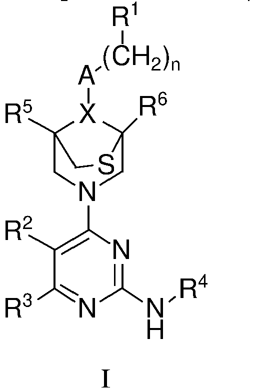 wherein,
X is selected from the group consisting of N and CR, wherein R is selected from the group consisting of hydrogen, deuterium, halogen, CN, hydroxy, CF₃, N(Rₒ)₂, substituted or unsubstituted C1-C4 alkyl, C1-C4 alkoxy, and substituted or unsubstituted C3-C6 cycloalkyl;
A is selected from the group consisting of bond, C=O, -SO₂-, -(C=O)NRₒ-; wherein Rₒ is H or C1-C4 alkyl;
R¹ is independently selected from hydrogen, deuterium, CN, N(Rₒ)₂, substituted or unsubstituted C1-C4 alkyl, substituted or unsubstituted C1-C6 alkoxy, substituted or unsubstituted C3-C6 cycloalkyl, substituted or unsubstituted C3-C6 heterocyclyl, substituted or unsubstituted aryl, substituted or unsubstituted 5-12 membered heteroaryl, substituted or unsubstituted aryl (C1-C6 alkyl), substituted or unsubstituted 5-12 membered heteroaryl (C1-C6 alkyl) and substituted or unsubstituted heterocyclyl (C1-C6 alkyl); wherein the "substituted or unsubstituted aryl (C1-C6 alkyl)" means that one or more hydrogen atoms of the aryl moiety and/or the alkyl moiety are substituted by substituents, "substituted or unsubstituted 5-12-membered heteroaryl (C1-C6 alkyl)" and "substituted or unsubstituted heterocyclyl(C1-C6 alkyl)" have similar meaning;
R² is selected from hydrogen, deuterium, C1-C4 alkyl, C3-C6 cycloalkyl, halogen and cyano, wherein the alkyl or cycloalkyl may be substituted by one or more fluorine atoms;
R³ is selected from hydrogen, deuterium and amino;
R ⁴ is 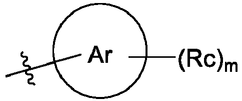 , wherein 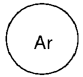 is selected from the group consisting of substituted or unsubstituted C6-C10 monocyclic or bicyclic aryl, and substituted or unsubstituted 5-12-membered monocyclic or bicyclic heteroaryl;
Rc is selected from the group consisting of halogen, CN, hydroxyl, amino, - COOH, -(CO)NR⁷R⁸, -(SO₂)NR⁷R⁸, -SO₂R⁷, -NR⁷COR⁸, -NR⁷SO₂R⁸, -(CR⁷R⁸)-R⁹, monosubstituted or disubstituted amino, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl;
R⁵ is selected from H, or C1-C4 alkyl;
R⁶ is selected from H, or C1-C4 alkyl;
R⁷, R⁸ and R⁹ are each independently selected from hydrogen, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl; or R⁷ and R⁸ together with the atoms to which they are attached form the corresponding 3-8 membered carbocyclic or heterocyclic ring;
n and q are each 0, 1 or 2;
m is each 0, 1, 2, 3 or 4; when m> 1, each Rc is independent from each other;
unless otherwise specified, the "substituted" means being substituted by one or more (e.g. 2, 3, 4, etc.) substituents selected from the group consisting of halogen, C1-C6 alkyl, halogenated C1-C6 alkyl, C1-C6 alkoxy, halogenated C1-C6 alkoxy, C3-C8 cycloalkyl, halogenated C3-C8 cycloalkyl, oxo, -CN, hydroxyl, amino, carboxyl, - COOH, -(CO)NH₂, or two substituents on a same atom together with the atom to form a C3-C6 cycloalkyl; and a group which is unsubstituted or substituted by one or more substituents, the group is selected from the group consisting of C6-C10 aryl, halogenated C6-C10 aryl, 5-10 membered heteroaryl with 1-3 heteroatoms selected from N, S and O, halogenated 5-10 membered heteroaryl with 1-3 heteroatoms selected from N, S and O, -(CO)NH(C1-C6 alkyl) and -(CO)N(C1-C6 alkyl)₂; while the substituent is selected from the group consisting of halogen, and C1-C6 alkoxy;
unless otherwise specified, the heteroaryl or heterocyclyl means that the ring atoms of the group contain 1, 2 or 3 heteroatoms selected from N, O and S.

In another preferred embodiment, X is N.

In another preferred embodiment, A is selected from the group consisting of C=O and -(C=O)NRₒ-, wherein Rₒ is H or C1-C4 alkyl.

In another preferred embodiment, R¹ is independently selected from CN, N(Rₒ)₂, substituted or unsubstituted C1-C4 alkyl, substituted or unsubstituted C1-C6 alkoxy, substituted or unsubstituted C3-C6 cycloalkyl, substituted or unsubstituted C3-C6 heterocyclyl, substituted or unsubstituted aryl, substituted or unsubstituted 5-12 membered heteroaryl, substituted or unsubstituted aryl(C1-C6 alkyl), substituted or unsubstituted 5-12 membered heteroaryl(C1-C6 alkyl) and substituted or unsubstituted heterocyclyl(C1-C6 alkyl).

In another preferred embodiment, R² is selected from hydrogen, halogen and cyano;
R³ is hydrogen.

In another preferred embodiment, R² is hydrogen or F.

In another preferred embodiment, Rc is selected from the group consisting of - (CO)NR⁷R⁸, -(SO₂)NR⁷R⁸, -SO₂R⁷, -NR⁷COR⁸, -NR⁷SO₂R⁸, -(CR⁷R⁸)-R⁹, monosubstituted or disubstituted amino, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl.

In another preferred embodiment, the 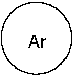 is selected from the group consisting of substituted or unsubstituted phenyl, and 5-7 membered heteroaryl.

In another preferred embodiment, the 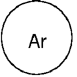 is a substituted or unsubstituted group selected from the group consisting of

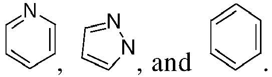

In another preferred embodiment, the Rc is selected from the group consisting of C1-C6 alkyl,

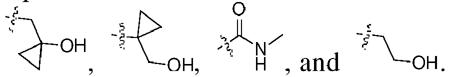

In another preferred embodiment, the compound of formula I is selected from the group consisting of

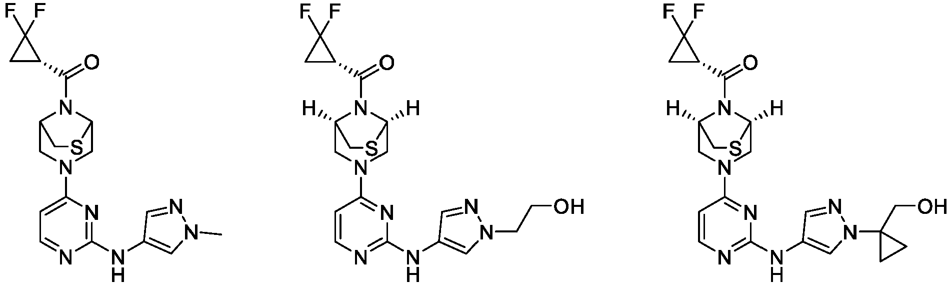

In a second aspect of the invention, a pharmaceutical composition is provided, which comprising (1) the compound, or the stereoisomer or tautomer, or the pharmaceutically acceptable salt, or the hydrate or solvate thereof of the first aspect of the present invention; and (2) a pharmaceutically acceptable carrier.

In a third aspect of the present invention, it provides a use of the compound, or the stereoisomer or tautomer, or the pharmaceutically acceptable salt, or the hydrate or solvate thereof of the first aspect of the present invention or the pharmaceutical composition of the second aspect for preparing a pharmaceutical composition for preventing and / or treating the disease or condition related to JAK kinase activity or expression level.

In another preferred embodiment, the disease or condition is selected from the group consisting of inflammation, autoimmune diseases, neuroinflammation, arthritis, rheumatoid arthritis, spondyloarthritis, systemic lupus erythematosus, lupus nephritis, gouty arthritis, pain, fever, pleuropulmonary sarcoidosis, silicosis, cardiovascular diseases, atherosclerosis, nodular myocarditis, myocarditis and cardiac reperfusion injury, cardiomyopathy, stroke, ischemia, reperfusion injury, cerebral edema, traumatic brain injury, neurodegenerative diseases, liver disease, inflammatory bowel disease, Crohn's disease, ulcerative colitis, nephritis, retinitis, retinopathy, macular degeneration, glaucoma, diabetes (type 1 and type 2), diabetic neuropathy, viral and bacterial infections, myalgia, endotoxic shock, toxic shock syndrome, autoimmune diseases, osteoporosis, multiple sclerosis, endometriosis, menstruation, vaginitis, candidiasis, cancer, fibrosis, obesity, muscular dystrophy, polymyositis, dermatomyositis, autoimmune hepatitis, primary biliary cirrhosis, primary sclerosing cholangitis, vitiligo, alopecia, Alzheimer's disease, skin flushing, eczema, psoriasis, atopic dermatitis and sunburn.

It should be understood that in the present invention, any of the technical features specifically described above and below (such as in the Example) can be combined with each other, thereby constituting new or preferred technical solutions. Limited by space, it will not be repeated here.

### DETAILED DESCRIPTION OF THE INVENTION

After long-term and in-depth research, the present inventors unexpectedly found a compound represented by formula I. The compounds have unexpected activity in regulating cytokines and/or interferons and are useful in the treatment of diseases mediated by cytokines and/or interferons. The inventors have completed the present invention based on this discovery.

### Definition

As used herein, the term "alkyl" includes a linear or branched alkyl. For example, C₁-C₈ alkyl represents a linear or branched chain alkyl having 1 to 8 carbon atoms, such as methyl, ethyl, propyl, isopropyl, butyl, isobutyl, tert-butyl, and the like.

As used herein, the term "alkenyl" includes a linear or branched alkenyl. For example, C₂-C₆ alkenyl refers to a linear or branched chain alkenyl having 2-6 carbon atoms, such as vinyl, allyl, 1-propenyl, isopropenyl, 1-butenyl, 2-butenyl, or the like.

As used herein, the term "alkynyl" includes a straight or branched chain alkynyl. For example, C₂-C₆ alkynyl refers to a linear or branched chain alkynyl with 2-6 carbon atoms, such as acetenyl, propinyl, butynyl, or the like.

As used herein, the term "C₃-C₁₀ cycloalkyl" refers to a cycloalkyl having 3-10 carbon atoms. It may be monocyclic, such as cyclopropyl, cyclobutyl, cyclopentyl, cyclohexyl, or the like. It can also be bicyclic, such as a bridged ring or a spiro ring.

As used herein, the term "C₁-C₈ alkylamino" refers to an amino substituted by C₁-C₈ alkyl, which may be mono- or disubstituted; for example, methylamino, ethylamino, propylamino, ipropylamino, butylamino, isobutylamino, tert-butylamino, dimethylamino, diethylamino, dipropylamino, diisopropylamino, dibutylamino, diisobutylamino, di-tert-butylamino, etc.

As used herein, the term "C₁-C₈ alkoxy" refers to a straight or branched chain alkoxy having 1-8 carbon atoms; e.g., methoxy, ethoxy, propoxy, isopropoxy, butoxy, isobutoxy, tert-butoxy, etc.

As used herein, the term "3-10 membered heterocycloalkyl having 1-3 heteroatoms selected from the group consisting of N, S and O" refers to saturated or partially saturated cyclic group having 3-10 atoms and 1-3 heteroatoms selected from N, S and O. It can be monocyclic, and it can also be bicyclic, such as a bridged ring or a spiro ring. Specific examples may be oxetanyl, azetidinyl, tetrahydro-2H-pyranyl, piperidinyl, tetrahydrofurfuryl, morpholinyl and pyrrolyl, etc..

As used herein, the term "C₆-C₁₀ aryl" refers to an aryl having 6-10 carbon atoms, e.g., phenyl or naphthyl and the like.

As used herein, the term "5-12 membered heteroaryl" refers to a cyclic aromatic group having 5-12 atoms and wherein 1-3 atoms are heteroatoms selected from N, S and O. It can be monocyclic, and it can also be fused. Specific examples may be pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furyl, thienyl, isoxazolyl, thiazolyl, oxazolyl, etc..

Unless specifically stated, the groups described in the present invention are "substituted or unsubstituted", the groups of the present invention can be substituted by substituents selected from the group consisting of halogen, cyano, nitro, hydroxyl, amino, C₁-C₆ alkyl-amino, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

As used herein, "halogen" or "halogen atom" refers to F, Cl, Br, and I. More preferably, the halogen or halogen atom is selected from F, Cl and Br. "Halogenated" means being substituted by an atom selected from F, Cl, Br, and I.

Unless otherwise specified, the structural formula described in the present invention is intended to include all isomeric forms (e. g., enantiomeric, diastereomeric, and geometric (or conformational) isomers): for example, R and S configurations of asymmetric centers, (Z) and (E) isomers of double bonds, etc. Thus, a single stereochemical isomer of the compound of the invention or a mixture of its enantiomers, diastereomers or geometric isomers (or conformational isomers) is within the scope of the invention.

As used herein, the term "tautomer" means that structural isomers with different energies can cross a low energy barrier and thus convert to each other. For example, proton tautomers (i.e. proton shift) include intertransformation through proton migration, such as 1H-indazole and 2H-indazole. Valence tautomers include interchange through some bonding electron recombination.

As used herein, the term "solvate" refers to a complex with specific proportion formed by the compound of the invention coordinates with a solvent molecule.

As used herein, the term "hydrate" refers to a complex formed by the coordination of a compound of the present invention with water.

The compounds of the present application can be prepared by a variety of synthetic methods well known to those skilled in the art, including the specific embodiments listed below, the embodiments formed by the combination of specific embodiments and other chemical synthesis methods, and equivalents well-known for those skilled in the art, preferred embodiments include, but are not limited to, the Examples of the present application.

The solvent used in this application is commercially available. Abbreviations used in this application are such as aq represents aqueous solution; HATU represents O-(7-Aza-1H-Benzotriazole-1-yl)-1,1,3,3-tetramethyluronium hexafluorophosphate; EDC represents N-(3-dimethylaminopropyl)-N'-ethylcarbodiimide hydrochloride; m-CPBA represents 3-chloroperoxybenzoic acid; eq represents equivalent; CDI represents carbonyldiimidazole ; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; Cbz represents benzyloxycarbonyl, an amino protecting group; Boc represents tert-butyloxycarbonyl, an amino protecting group; HOAc represents acetic acid; NaCNBH₃ represents sodium cyanoborohydride; r.t. represents room temperature; THF represents tetrahydrofuran; TFA represents trifluoroacetic acid; DIPEA represents diisopropylethylamine; Boc₂O represents di-tert-butyldicarbonate; LDA represents lithium diisopropylamide.

Compounds were named manually or by ChemDraw^{®} software, and commercially available compounds were named by supplier catalog.

### Pharmaceutical composition and method of administration

Since the compound of the present invention has excellent inhibitory activity of cytokines and/or interferons, the compound of the present invention and its various crystal forms, pharmaceutically acceptable inorganic or organic salts, hydrates or solvates, and the pharmaceutical composition containing the compound of the present invention as the main active ingredient can be used to prevent and/or treat (stabilize, alleviate or cure) a variety of autoimmune and inflammation-related diseases, including systemic lupus erythematosus, inflammatory bowel disease, psoriasis, rheumatoid arthritis, rheumatoid arthritis, etc..

The pharmaceutical composition of the present invention comprises a safe and effective amount of the compound of the present invention and a pharmaceutically acceptable excipient or carrier. Wherein "safe and effective amount" refers to the amount of compound is sufficient to significantly improve the condition, not to produce severe side effects. Typically, the pharmaceutical composition contains 1-2000 mg of the compound / dosage of the present invention, and preferrably contains 1-200mg of the compound / dosage of the present invention. Preferably, "one dosage" is a capsule or a pill.

"Pharmaceutically acceptable carrier" refers to one or more compatible solid or liquid filler or gel substances, which are suitable for human use, and must be sufficiently pure and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

There is no special limitation of administration mode for the compound or pharmaceutical compositions of the present invention, and the representative administration mode includes (but is not limited to) oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, such as ethanol, isopropanol, ethyl carbonate, ethyl acetate, propylene glycol, 1,3-butanediol, dimethylformamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The compounds of the present invention may be administered alone or in combination with other pharmaceutically acceptable therapeutic agents.

When administered in combination, the pharmaceutical composition also includes one or more (2, 3, 4, or more) other pharmaceutically acceptable therapeutic agents. One or more (2, 3, 4, or more) of the other pharmaceutically acceptable therapeutic agents may be used simultaneously, separately or sequentially with the compounds of the present invention for the prevention and/or treatment of diseases related to cytokine and/or interferon.

When the pharmaceutical composition is used, a safe and effective amount of the compound of the present invention is applied to a mammal (such as a human) in need of treatment, wherein the dose is considered as a pharmaceutically effective dose. For a person weighing 60kg, the daily dose is usually 1 to 2000mg, preferably 1 to 500mg. Of course, the particular dose should also depend on various factors, such as the route of administration, patient healthy status, which are well within the skills of an experienced physician.

The present invention will be further illustrated below with reference to the specific examples. It should be understood that these examples are only to illustrate the invention but not to limit the scope of the invention. The experimental methods in which conditions have not been specified in the following examples are usually based on conventional conditions, or according to mature techniques known in the field.

### Example 1

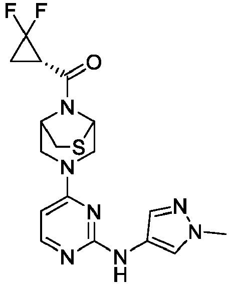

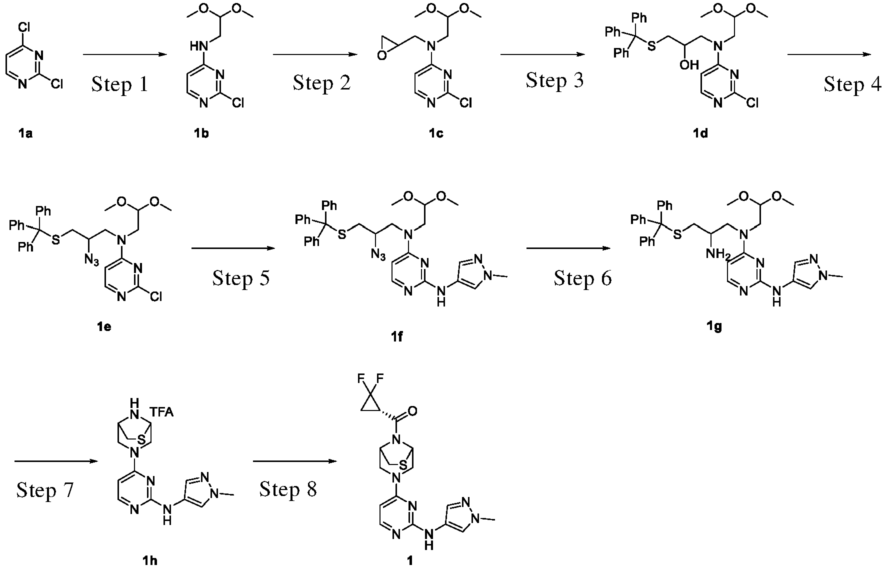

### Step 1:

1a (5.00 g, 34.00 mmol), 2,2-dimethoxyethylamine (3.90 g, 37.00 mmol), and triethylamine (6.80 g, 74.00 mmol) were dissolved in acetonitrile (70 mL), stirred at room temperature for 16 hours. The reaction was concentrated under reduced pressure to obtain a residue, which was dissolved in ethyl acetate (100 mL), washed with water (100 mL × 3), dried over anhydrous sodium sulfate, filtered, and concentrated under reduced pressure to obtain a residue. The residue was slurried with petroleum ether: diethyl ether (40 mL:10 mL) to obtain **1b** (6.00 g), yield: 82%.

MS-ESI calculated value [M+1] ⁺ 218, found 218.

### Step 2:

**1b** (4.00 g, 18.38 mmol) was dissolved in N,N-dimethylformamide (40 mL), and 60% sodium hydride (885 mg, 22.13 mmol) was added to the reaction at 0 °C. After stirring for 10 minutes, epichlorohydrin (1.90 g, 20.54 mmol) was added to the reaction system, and the reaction was carried out at room temperature for 3 hours, quenched by adding water (200 mL), and then extracted with ethyl acetate (400 mL x 1). The organic phase was washed with water (200 mL × 3), dried over anhydrous sodium sulfate, and concentrated under reduced pressure to obtain the crude product **1c** (5.00 g).

MS-ESI calculated value [M+1] ⁺ 274, found 274.

### Step 3:

Triphenylmethanethiol (5.00 g, 18 mmol) was dissolved in tetrahydrofuran (40 mL), sodium hydride (60%, 800 mg, 20.00 mmol) was added to the reaction at 0 °C and stirred for 10 minutes, then the crude product **1c** (5.00 g, 18.38 mmol) was added, and the reaction system was kept at 0 °C for 20 minutes. After the reaction was completed, water (200 mL) was added to quench the reation and ethyl acetate (400 mL) was added, then separate organic phase. The organic phase was washed with water (200 mL × 3), dried over anhydrous sodium sulfate, filtered, and concentrated under reduced pressure to obtain a residue. The residue was purified by reverse-phase column chromatography (acetonitrile: water = 0-100%) to obtain **1d** (3.00 g), two-step yield: 30%.

MS-ESI calculated value [M+1] ⁺ 550, found 550.

### Step 4:

**1d** (3.00 g, 5.50 mmol), triphenylphosphine (2.10 g, 8.20 mmol), diphenylphosphoryl azide (2.20 g, 8.20 mmol) were dissolved in tetrahydrofuran (40 mL). Diisopropyl azodicarboxylate (1.70 g, 5.50 mmol) was added dropwise to the reaction system at 0 °C. After the addition was completed, the mixture was stirred at room temperature for 2 hours. The reaction system was quenched with water (100 mL), and extracted with ethyl acetate (200 mL × 1). The organic phase was washed with water (100 mL × 2), dried over anhydrous sodium sulfate, filtered, and concentrated under reduced pressure to obtain a residue. The residue was purified by reverse-phase column chromatography (acetonitrile:water=0-100%) to obtain **1e** (1.80 g), yield: 58%.

### Step 5:

**1e** (1.80 g, 3.10 mmol), and 1-methyl-1*H*-pyrazol-4-amine (456 mg, 4.70 mmol) were dissolved in n-butanol (20 mL) and stirried at 120 °C for 2 hours. Most of the solvent n-butanol was removed by concentration under reduced pressure, and the residue was purified by reverse-phase column chromatography (acetonitrile: water = 0-100%) to obtain **If** (1.00 g), yield: 50%.

MS-ESI calculated value [M+1] ⁺ 636, found 636.

### Step 6

**If** (450 mg, 0.70 mmol), and triphenylphosphine (204 mg, 0.78 mmol) were dissolved in a mixed solvent of tetrahydrofuran (8 mL) and water (2 mL), and the reaction was heated to 60 °C to continued for 2 hours, then cooled to room temperature to obtain a reaction solution (10 mL) of **1 g**, which was directly used in the next step.

MS-ESI calculated value [M+1] ⁺ 610, found 610.

### Step 7

Trifluoroacetic acid (10 mL) was added to the reaction solution (10 mL, 0.70 mmol) of **1 g**, and the reaction solution was warmed to 60 °C and stirred for 2 hours. The reaction solution was concentrated under reduced pressure to obtain a residue, which was purified by reverse phase column chromatography (acetonitrile:water = 0-100%) to obtain **1h** (150 mg), two-step yield: 55%.

### Step 8

**1h** (150 mg, 0.50 mmol), (*S*)-2,2-difluorocyclopropanecarboxylic acid(72 mg, 0.60 mmol), 2-(7-azabenzotriazol-1-yl)-N,N,N',N'-tetramethyluronium hexafluorophosphate (285 mg, 0.75 mmol), and triethylamine (101 mg, 1 mmol) were dissolved in N,N-dimethylformamide (3 mL), and stirred at room temperature for 30 minutes. The reaction system was quenched by adding water (30 mL), then extracted with ethyl acetate (50 mL × 1). The organic phase was washed with water (20 mL × 2), and concentrated under reduced pressure to obtain a residue, which was purified by high performance liquid chromatography to obtain **1** (18 mg), yield: 10%.

MS-ESI calculated value [M+1] ⁺ 408, found 408.

¹H NMR(400 MHz, DMSO-*d₆*) δ 8.90 (brs, 1H), 7.93 (t, J=6.0 Hz, 1H), 7.74 (brs, 1H), 7.43 (s, 1H), 6.15-5.97 (m, 2H), 5.21-5.11 (m, 1H), 4.42-4.22 (m, 2H), 3.79 (s, 3H), 3.35-3.16 (m, 3H), 3.06-2.87(m, 2H), 2.01-1.98(m, 2H).

### Example 2

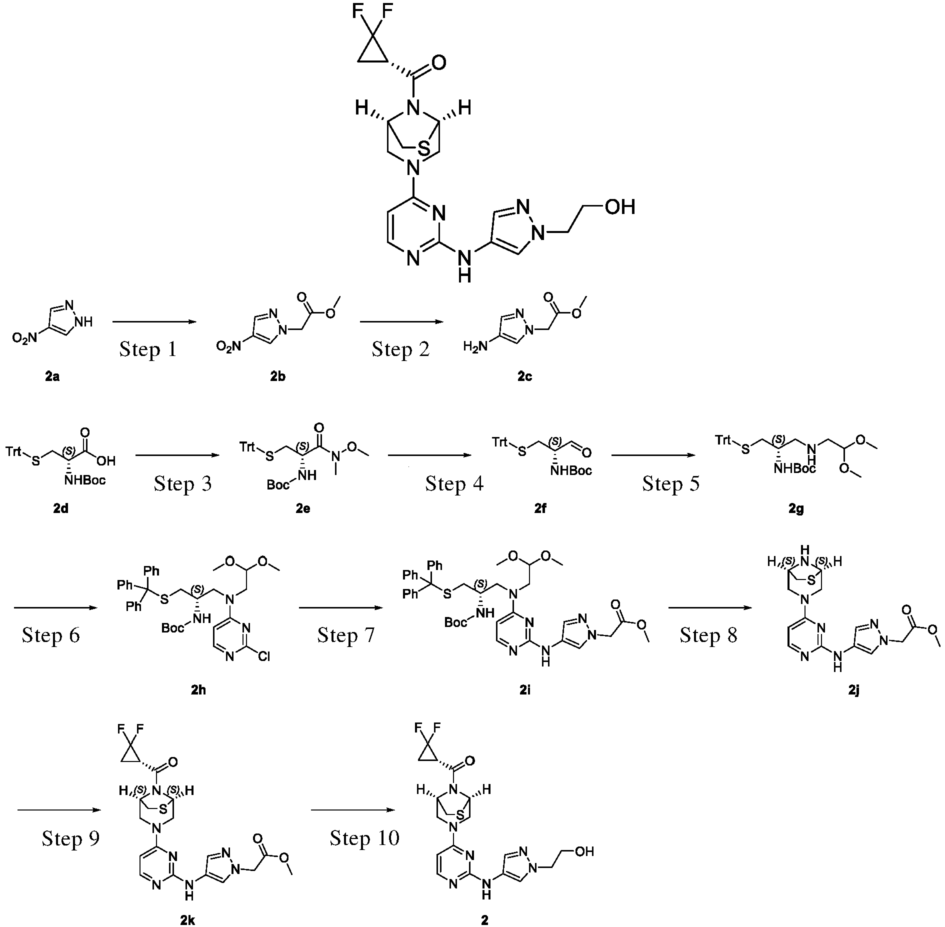

### Step 1:

Under nitrogen protection, compound **2a** (6.00 g, 53.10 mmol) was dissolved in N,N-dimethylformamide (50 mL), and 60% sodium hydride (2.50 g, 63.72 mmol) was added in batches under an ice bath. Methyl bromoacetate (6.00 mL, 63.72 mmol) was added after reacting for 30 minutes, and the reaction was continued for 2 hours under an ice bath. The reaction system was quenched by adding water (200 mL) under an ice bath, and extracted with ethyl acetate (200 mL × 2). The organic phase was successively washed with saturated brine (200 mL × 1), dried over anhydrous sodium sulfate, filtered, and concentrated under reduced pressure to obtain a residue. The residue was purified by column chromatography (ethyl acetate:petroleum ether=0-100%) to obtain compound **2b** (8.80 g), yield: 89%.

MS-ESI calculated value [M+H] ⁺ 186, found 186.

### Step 2:

**2b** (8.80 g, 45.57 mmol) was dissolved in methanol (80 mL), and 10% wet palladium on carbon (800 mg) was added, then the system was replaced with hydrogen (15 psi), and reacted overnight at room temperature. The reaction solution was filtered through celite, and the filtrate was concentrated under reduced pressure to obtain **2c** (6.20 g), yield: 84%.

MS-ESI calculated value [M+H] ⁺ 156, found 156.

### Step 3:

Compound **2d** (50.00 g, 0.11 mol), N,O-dimethylhydroxylamine hydrochloride (12.60 g, 0.13 mol) and triethylamine (32.70 g, 0.13 mol) were dissolved in dichloromethane (500 mL) under nitrogen protection, then 1-ethyl-(3-dimethylaminopropyl)carbodiimide hydrochloride (24.80 g, 0.13 mol) and 1-hydroxybenzotriazole (17.50 g) , 0.13 mol) were added to the solution, and the reaction solution was reacted at room temperature overnight. The reaction solution was diluted with water (400 mL), and extracted with dichloromethane (200 mL × 2). The organic phase was washed successively with saturated brine (300 mL × 1), dried over anhydrous sodium sulfate, filtered, and concentrated under reduced pressure to obtain a residue. The residue was purified by column chromatography (ethyl acetate: petroleum ether = 0-100%) to obtain compound **2e** (52.10 g), yield: 95%.

MS-ESI calculated value [M+H] ⁺ 507, found 507.

### Step 4:

Compound **2e** (20.00 g, 39.53 mmol) was dissolved in ultra-dry tetrahydrofuran (200 mL) under nitrogen protection, and cooled to -78 °C. Lithium aluminum hydride (1.80 g, 47.43 mmol) was added in batches, and the reaction was continued for 2 hours. The temperature was kept at -78°C and 0.5M diluted hydrochloric acid (50 mL) was added to quench the reaction, and extracted with ethyl acetate (100 mL × 2). The organic phase was washed with saturated brine (100 mL × 1), dried over anhydrous sodium sulfate, filtered and concentrated under reduced pressure to obtain crude product **2f** (19.50 g).

### Step 5:

Under nitrogen protection, the crude product **2f** (19.50 g, 39.53 mmol) and 2,2-dimethoxyethylamine (13.7 g, 130.58 mmol) were dissolved in anhydrous methanol (200 mL) and cooled to 0 °C after reacted for 30 minutes. Sodium cyanoborohydride (3.20 g, 47.44 mmol) was added in batches, then cooled to room temperature overnight. The reaction solution was concentrated under reduced pressure to obtain a residue, which was purified by column chromatography (ethyl acetate: petroleum ether = 0-100%) to obtain compound **2g** (9.20 g), yield: 43%.

MS-ESI calculated value [M+H] ⁺ 537, found 537.

¹H NMR(400 MHz, CDCl₃) δ 7.41-7.39 (m, 6H), 7.30-7.26 (m, 6H), 7.22-7.18 (m, 3H), 4.74-4.66 (brs, 1H), 4.36 (t, J = 5.6 Hz, 1H), 3.67-3.62 (m, 1H), 3.34 (s, 6H), 2.67-2.61 (m, 3H), 2.55-2.41 (m, 1H), 2.24-2.04 (m, 2H), 1.42 (s, 9H).

### Step 6

**2g** (9.20 g, 16.17 mmol), triethylamine (4.80 mL, 34.32 mmol) and 2,4-dichloropyrimidine (3.10 g, 20.60 mmol) were dissolved in ethanol (100 mL), heated to 70 °C and reacted overnight. The reaction solution was naturally cooled to room temperature, concentrated under reduced pressure to obtain a residue, which was purified by column chromatography (ethyl acetate: petroleum ether = 0-100%) to obtain compound **2h** (7.10 g), yield: 64%.

MS-ESI calculated value [M+H] ⁺ 649, found 649.

### Step 7

**2h** (3.00 g, 4.63 mmol) and **2c** (861 mg, 5.56 mmol) were dissolved in isopropanol (20 mL), heated to 130 °C and reacted for 1 hour. The reaction solution was cooled to room temperature and concentrated under reduced pressure to obtain a residue, which was purified by reverse phase column chromatography (acetonitrile: water = 0-100%) to obtain compound **2i** (3.10 g), yield: 86%.

MS-ESI calculated value [M+1] ⁺ 768, found 768.

### Step 8

Compound **2i** (3.10 g, 4.04 mmol) was dissolved in a mixed solvent of trifluoroacetic acid (30 mL) and water (10 mL), heated to 60 °C and reacted for 1 hour. The reaction solution was cooled to room temperature and concentrated under reduced pressure to obtain a residue, which was purified by reverse phase column chromatography (acetonitrile: water = 0-100%) to obtain **2j** (1.30 g), yield: 86%.

MS-ESI calculated value [M+1] ⁺ 362, found 362.

### Step 9

Compound **2j** (400 mg, 1.11 mmol), (S)-2,2-difluorocyclopropanecarboxylic acid (162 mg, 1.33 mmol) and triethylamine (448 mg, 4.44 mmol) were dissolved in N,N-dimethylformamide (3 mL), 2-(7-azabenzotriazol-1-yl)-N,N,N',N'-tetramethyluronium hexafluorophosphate (634 mg, 1.67 mmol) was added to the reaction system and reacted at room temperature for 30 minutes. The reaction solution was concentrated under reduced pressure to obtain a residue, which was purified by preparative HPLC to obtain compound **2k** (18 mg), yield: 43%.

MS-ESI calculated value [M+1] ⁺466, found 466.

### Step 10

Under nitrogen protection, **2k** (570 mg, 1.23 mmol) was dissolved in a mixed solvent of tetrahydrofuran (2 mL) and ethanol (5 mL), cooled to 0 °C, and a solution of 2M lithium borohydride in tetrahydrofuran (3.10 mL, 6.13 mmol) was added dropwise. The reaction solution was naturally warmed to room temperature and reacted for 2 hours. The reaction was quenched with saturated ammonium chloride (1 mL), and concentrated under reduced pressure. The residue was sequentially purified by reverse-phase column chromatography (acetonitrile:water = 0-100%) and preparative HPLC to obtain compound **2** (80 mg), yield: 15%.

MS-ESI calculated value [M+1] ⁺ 438, found 438.

¹H NMR(400 MHz, DMSO-*d₆*) δ 8.90 (s, 1H), 8.14 (s, 1H), 7.94 (d, J=6.0 Hz, 1H), 7.81 (s, 1H), 7.45 (s, 1H), 6.15-5.93 (m, 2H), 5.21-5.09 (m, 1H), 4.89-4.77 (m, 1H), 4.46-4.22 (m, 1H), 4.08 (t, J=5.2 Hz, 2H), 3.70 (t, J=5.2 Hz, 2H), 3.19-3.16 (m, 2H), 3.02-2.85 (m, 3H), 2.02-1.98 (m, 2H).

### Example 3

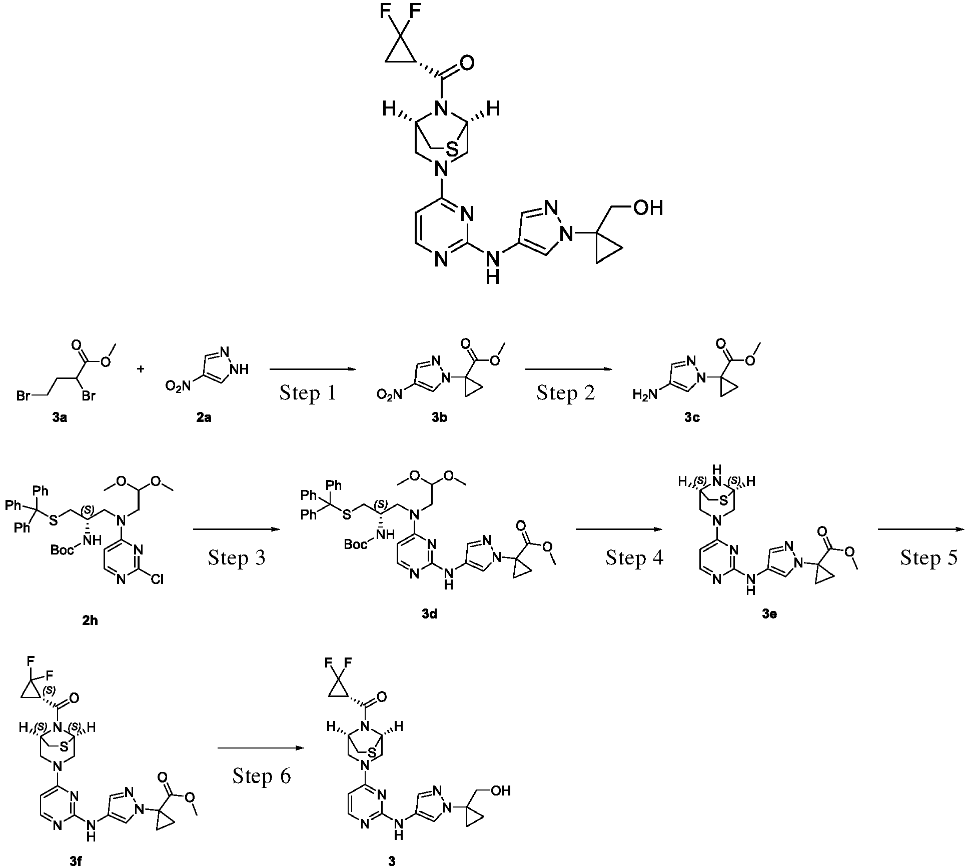

### Step 1:

60% sodium hydride (142 mg, 3.54 mmol) was added to a solution of **2a** (200 mg, 1.77 mmol) in N,N-dimethylformamide (3 mL) under nitrogen protection in an ice bath. After reacted for 30 minutes, **3a** (460 mg, 1.77 mmol) was added to the system, and the reaction was carried out at room temperature overnight. The reaction solution was directly purified by reverse-phase column chromatography (acetonitrile: water = 0-100%) to obtain compound **3b** (240 mg), yield: 64%.

MS-ESI calculated value [M+H] ⁺ 212, found 212.

¹H NMR(400 MHz, CDCl₃) δ 8.28 (s, 1H), 8.08 (s, 1H), 3.73 (s, 3H), 1.93-1.90 (m, 2H), 1.73-1.70 (m, 2H).

### Step 2:

Under nitrogen protection, 10% wet palladium on carbon (10 mg) was added to a solution of **3b** (130 mg, 0.62 mmol) in methanol (3 mL), and the reaction system was reacted at room temperature for 3 hours. The reaction system was filtered through celite and concentrated under reduced pressure to obtain crude **3c** (110 mg).

MS-ESI calculated value [M+H] ⁺ 182, found 182.

### Step 3:

**2h** (3.00 g, 4.63 mmol) and **3c** (1.01 g, 5.56 mmol) were dissolved in isopropanol (20 mL), heated to 130 °C to react for 1 hour. The reaction solution was directly concentrated under reduced pressure, and the residue was purified by reverse-phase column chromatography (acetonitrile: water = 0-100%) to obtain **3d** (3.30 g), yield: 82%.

MS-ESI calculated value [M+1] ⁺ 694, found 694.

### Step 4:

Compound **3d** (3.30 g, 3.98 mmol) was dissolved in a mixed system of trifluoroacetic acid (30 mL) and water (10 mL), heated to 60 °C and reacted for 1 hour. The reaction solution was concentrated under reduced pressure to obtain a residue, which was purified by reverse-phase column chromatography (acetonitrile: water = 0-100%) to obtain **3e** (1.10 g), yield: 72%.

MS-ESI calculated value [M+1] ⁺ 387, found 387.

### Step 5:

**3e** (60 mg, 0.15 mmol), (S)-2,2-difluorocyclopropanecarboxylic acid (18 mg, 0.15 mmol) and triethylamine (45 mg, 0.15 mmol) were dissolved in N,N-dimethylformamide (3 mL), 2-(7-azabenzotriazol-1-yl)-N,N,N',N'-tetramethyluronium hexafluorophosphate (87 mg, 0.23 mmol) was added to the reaction system and reacted at room temperature for 30 minutes. The reaction solution was directly purified by preparative HPLC to obtain compound **3f** (15 mg), yield: 20%.

MS-ESI calculated value [M+1] ⁺ 491, found 491.

### Step 6

**3f** (15 mg, 0.03 mmol) was dissolved in methanol (1 mL), and sodium borohydride (3 mg, 0.09 mmol) and lithium chloride (4 mg, 0.09 mmol) were added at 0 °C under nitrogen protection, and reacted at room temperature for 2 hours. The reaction solution was quenched with saturated ammonium chloride solution (1 mL), concentrated under reduced pressure to obtain a residue which was sequentially purified by reverse-phase column chromatography and preparative HPLC to obtain compound **3** (2 mg), yield: 12% .

MS-ESI calculated value [M+1] ⁺ 463, found 463.

¹H NMR(400 MHz, CDCl₃) δ 7.92 (d, *J* = 8.0 Hz, 1H), 7.73 (s, 1H), 7.62 (s, 1H), 6.03 (brs, 1H), 5.96 (d, *J* = 8.0 Hz, 1H), 5.56-5.51 (m, 1H), 5.38-5.26 (m, 1H), 4.86-4.76 (m, 1H), 3.77 (s, 2H), 3.41-3.31 (m, 1H), 3.18-2.94 (m, 2H), 2.68-2.15 (m, 4H), 1.96-1.76 (m, 2H), 1.32-1.25 (m, 2H), 1.15-1.08 (m, 2H).

### Biological activity test

### Kinase experiment method

The in vitro inhibitory effect of compounds on JAK1, JAK2, JAK3 or TYK2 kinase was detected by Caliper mobility shift assay. Compounds to be tested were dissolved in DMSO, and prepared into 10 mM stock solutions. Stock solutions of the compounds were prepared into 50× working solutions (10 concentrations in total) through gradient dilution using DMSO, and each concentration of working solution was transferred to an Echo^{®} master plate. The Echo^{®} non-contact nanoliter sonic pipetting system was used to transfer 5 µL of compound solution or DMSO of the corresponding concentration from the master plate to the 384-well reaction plate. Then, 10 µL of 2.5× kinase solution was added to the 384-well reaction plate. After 10 minutes of incubation at room temperature, 10 µL of mixed solution of 2.5× FAMlabeled polypeptide and ATP (final ATP concentration was 1 mM) was added. 30 µL of stop reagent was added, detected on Caliper, and the conversion value was calculated, i.e. the height of the product peak divided by the sum of the heights of the substrate and product peaks. The percent inhibition of the kinase by the compounds was calculated using the formula below, and the IC₅₀ value was fitted by using XLFit 5.4.0.8.

Percent inhibition = (max-conversion)/(max-min) × 100, wherein "max" represents the conversion reading for the DMSO control and "min" represents the conversion reading for the low value control. The specific test results are shown in Table-1.

**Table-1 Test results of compounds of the present invention against JAK family kinases**

| Example | TYK2 IC₅₀ (nM) | JAK1 IC₅₀ (nM) | JAK2 IC₅₀ (nM) | JAK3 IC₅₀ (nM) |
|---|---|---|---|---|
| Example 1 | 4.2 | 32 | 27 | 3473 |
| Example 2 | 1.7 | 10 | 1.4 | 2269 |
| Example 3 | 2.9 | 107 | N/T | N/T |

| | | | | |
|---|---|---|---|---|
| N/T: No data yet Note: Activity data were obtained at 1 mM ATP concentration. | | | | |

### Cell experiment method

In murine B lymphocytes BaF3, recombinant genes (including TEL and human JAK1, TYK2, JAK2, JAK3 kinase domains) were transferred by using genetic engineering technology, in which TEL could promote phosphorylation of TEL-JAK1 or TEL-TYK2 kinase dimer and continuously activate, thus allowing cells to grow dependent on the activity of this recombinant kinase. If possessing JAK1 or TYK2 kinase inhibitory activity, the compound can cause cell death by inhibiting the kinase activity. CellTiter-Glo method was used to detect the proliferation of genetically engineered cell lines Ba/F3-TEL-JAK1, Ba/F3-TEL-TYK2, Ba/F3-TEL-JAK2, and Ba/F3-TEL-JAK3 which was cultured in vitro. Graphpad 7.0 was used to fit curves and IC₅₀ values were calculated.

**Table-2 Inhibitory activity of the compounds of the present invention on the proliferation of murine B lymphocyte BaF3 cells**

| Example | Ba/F3-TEL-TYK2 cells IC₅₀ (nM) | Ba/F3-TEL-JAK1 cells IC₅₀ (nM) | Ba/F3-TEL-JAK2 cells IC₅₀ (nM) | Ba/F3-TEL-JAK3 cells IC₅₀ (nM) |
|---|---|---|---|---|
| Example 1 | 111 | 31 | 87 | 3689 |
| Example 2 | 192 | 314 | 173 | 8637 |
| Example 3 | 556 | 56 | 427 | > 10000 |

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of the structure shown in formula I, or an enantiomer, or a pharmaceutically acceptable salt thereof: 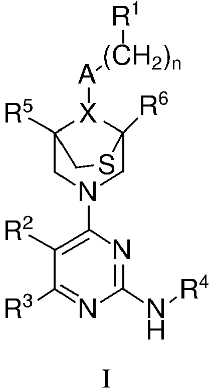 wherein,
X is selected from the group consisting of N and CR, wherein R is selected from the group consisting of hydrogen, deuterium, halogen, CN, hydroxy, CF₃, N(Rₒ)₂, substituted or unsubstituted C1-C4 alkyl, C1-C4 alkoxy, and substituted or unsubstituted C3-C6 cycloalkyl;
A is selected from the group consisting of bond, C=O, -SO₂-, -(C=O)NRₒ-; wherein Rₒ is H or C1-C4 alkyl;
R¹ is independently selected from hydrogen, deuterium, CN, N(Rₒ)₂, substituted or unsubstituted C1-C4 alkyl, substituted or unsubstituted C1-C6 alkoxy, substituted or unsubstituted C3-C6 cycloalkyl, substituted or unsubstituted C3-C6 heterocyclyl, substituted or unsubstituted aryl, substituted or unsubstituted 5-12 membered heteroaryl, substituted or unsubstituted aryl (C1-C6 alkyl), substituted or unsubstituted 5-12 membered heteroaryl (C1-C6 alkyl) and substituted or unsubstituted heterocyclyl(C1-C6 alkyl);
R² is selected from hydrogen, deuterium, C1-C4 alkyl, C3-C6 cycloalkyl, halogen and cyano, wherein the alkyl or cycloalkyl may be substituted by one or more fluorine atoms;
R³ is selected from hydrogen, deuterium and amino;
R⁴ is 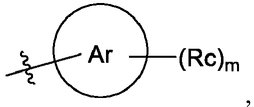 wherein 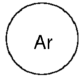 is selected from the group consisting of substituted or unsubstituted C6-C10 monocyclic or bicyclic aryl, and substituted or unsubstituted 5-12-membered monocyclic or bicyclic heteroaryl;
Rc is selected from the group consisting of halogen, CN, hydroxyl, amino, - COOH, -(CO)NR⁷R⁸, -(SO₂)NR⁷R⁸, -SO₂R⁷, -NR⁷COR⁸, -NR⁷SO₂R⁸, -(CR⁷R⁸)-R⁹, monosubstituted or disubstituted amino, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl;
R⁵ is selected from H, or C1-C4 alkyl;
R⁶ is selected from H, or C1-C4 alkyl;
R⁷, R⁸ and R⁹ are each independently selected from hydrogen, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl; or R⁷ and R⁸ together with the atoms to which they are attached form the corresponding 3-8 membered carbocyclic or heterocyclic ring;
n and q are each 0, 1 or 2;
m is each 0, 1, 2, 3 or 4; when m>1, each Rc is independent from each other;
unless otherwise specified, the "substituted" means being substituted by one or more (e.g. 2, 3, 4, etc.) substituents selected from the group consisting of halogen, C1-C6 alkyl, halogenated C1-C6 alkyl, C1-C6 alkoxy, halogenated C1-C6 alkoxy, C3-C8 cycloalkyl, halogenated C3-C8 cycloalkyl, oxo, -CN, hydroxyl, amino, carboxyl, - COOH, -(CO)NH₂, or two substituents on a same atom together with the atom to form a C3-C6 cycloalkyl; and a group which is unsubstituted or substituted by one or more substituents, the group is selected from the group consisting of C6-C10 aryl, halogenated C6-C10 aryl, 5-10 membered heteroaryl with 1-3 heteroatoms selected from N, S and O, halogenated 5-10 membered heteroaryl with 1-3 heteroatoms selected from N, S and O, -(CO)NH(C1-C6 alkyl) and -(CO)N(C1-C6 alkyl)₂; while the substituent is selected from the group consisting of halogen, and C1-C6 alkoxy;
unless otherwise specified, the heteroaryl or heterocyclyl means that the ring atoms of the group contain 1, 2 or 3 heteroatoms selected from N, O and S.

2. The compound of claim 1, wherein X is N.

3. The compound of claim 1, wherein A is selected from the group consisting of C=O and -(C=O)NRₒ-, wherein Rₒ is H or C1-C4 alkyl.

4. The compound of claim 1, wherein R¹ is independently selected from CN, N(Rₒ)₂, substituted or unsubstituted C1-C4 alkyl, substituted or unsubstituted C1-C6 alkoxy, substituted or unsubstituted C3-C6 cycloalkyl, substituted or unsubstituted C3-C6 heterocyclyl, substituted or unsubstituted aryl, substituted or unsubstituted 5-12 membered heteroaryl, substituted or unsubstituted aryl (C1-C6 alkyl), substituted or unsubstituted 5-12 membered heteroaryl (C1-C6 alkyl) and substituted or unsubstituted heterocyclyl (C1-C6 alkyl);

5. The compound of claim 1, wherein R² is selected from the group consisting of hydrogen, halogen and cyano;
R³ is hydrogen.

6. The compound of claim 1, wherein Rc is selected from the group consisting of -(CO)NR⁷R⁸, -(SO₂)NR⁷R⁸, -SO₂R⁷, -NR⁷COR⁸, -NR⁷SO₂R⁸, -(CR⁷R⁸)-R⁹, monosubstituted or disubstituted amino, substituted or unsubstituted C1-C6 alkyl, substituted or unsubstituted C1-C6 alkoxy, substituted or unsubstituted C3-C6 cycloalkyl, and substituted or unsubstituted 5-12 membered heterocyclyl.

7. The compound of claim 1, wherein the compound of formula I is selected from the group consisting of

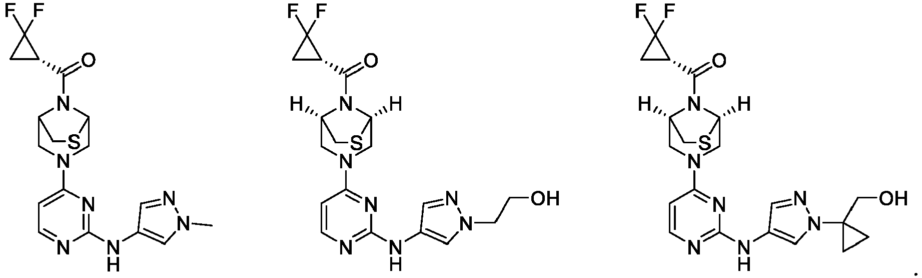

8. A pharmaceutical composition comprising (1) the compound of any one of claims 1-7, or the stereoisomer or tautomer thereof, or the pharmaceutically acceptable salt, or the hydrate or a solvate thereof; and (2) a pharmaceutically acceptable carrier.

9. A use of the compound, or the stereoisomer or tautomer, or the pharmaceutically acceptable salt, or the hydrate or solvate thereof of any one of claims 1 to 7, or the pharmaceutical composition of claim 8 for preparing a pharmaceutical composition for preventing and / or treating the disease or condition related to JAK kinase activity or expression level.

10. The use of claim 9, wherein the disease or condition is selected from the group consisting of inflammation, autoimmune diseases, neuroinflammation, arthritis, rheumatoid arthritis, spondyloarthritis, systemic lupus erythematosus, lupus nephritis, gouty arthritis, pain, fever, pleuropulmonary sarcoidosis, silicosis, cardiovascular disease, atherosclerosis, nodular myocarditis, myocarditis and cardiac reperfusion injury, cardiomyopathy, stroke, ischemia, reperfusion injury, cerebral edema, traumatic brain injury, neurodegenerative diseases, liver disease, inflammatory bowel disease, Crohn's disease, ulcerative colitis, nephritis, retinitis, retinopathy, macular degeneration, glaucoma, diabetes (type 1 and type 2), diabetic neuropathy, viral and bacterial infections, myalgia, endotoxic shock, toxic shock syndrome, autoimmune diseases, osteoporosis, multiple sclerosis, endometriosis, menstruation, vaginitis, candidiasis, cancer, fibrosis, obesity, muscular dystrophy, polymyositis, dermatomyositis, autoimmune hepatitis, primary biliary cirrhosis, primary sclerosing cholangitis, vitiligo, alopecia, Alzheimer's disease, skin flushing, eczema, psoriasis, atopic dermatitis and sunburn.
